(19) Europäisches Patentamt
European Patent Office
Office européen des brevets

(11) Veröffentlichungsnummer: **0 473 053 A1**

(12) **EUROPÄISCHE PATENTANMELDUNG**

(21) Anmeldenummer: **91114005.1**

(51) Int. Cl.5: **C07C 31/38**, C07C 29/44

(22) Anmeldetag: **21.08.91**

(30) Priorität: **24.08.90 DE 4026712**

(43) Veröffentlichungstag der Anmeldung:
**04.03.92 Patentblatt 92/10**

(84) Benannte Vertragsstaaten:
**BE CH DE FR GB IT LI NL**

(71) Anmelder: **HOECHST AKTIENGESELLSCHAFT**
**Postfach 80 03 20**
**W-6230 Frankfurt am Main 80(DE)**

(72) Erfinder: **Knaup, Wolfgang, Dr.**
**Kanalstrasse 3**
**W-8269 Burgkirchen(DE)**

(54) **Verfahren zur Herstellung von tertiären fluorhaltigen Alkoholen.**

(57) Nach dem beschriebenen Verfahren werden tertiäre Fluoralkohole der nachstehenden Formel hergestellt

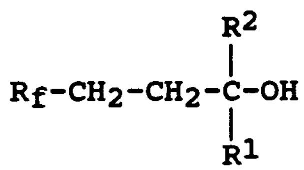

worin $R_f$ ein Perfluoralkylrest mit 4 bis 20 C-Atomen, $R^1$ $CH_3$ und $R^2$ $CH_3$ oder $C_2H_5$ ist, durch Umsetzung von Perfluoralkylethylenen der Formel $R_f\text{-}CH=CH_2$, worin $R_f$ die angegebene Bedeutung hat, mit überschüssigem Isopropanol oder überschüssigem sec.-Butanol in Gegenwart von Radikalinitiatoren.

EP 0 473 053 A1

Die Erfindung betrifft ein Verfahren zur Herstellung von tertiären fluorhaltigen Alkoholen der nachstehenden Formel 1

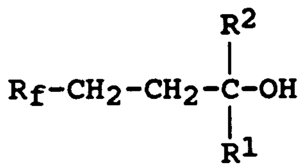

(1)

worin $R_f$ ein Perfluoralkylrest mit 4 bis 20 C-Atomen ist, $R^1$ $CH_3$ ist und $R^2$ $CH_3$ oder $C_2H_5$ ist.

Aus der US-Patentschrift 3,532,659 sind primäre und sekundäre fluorhaltige Alkohole der nachstehenden Formel bekannt

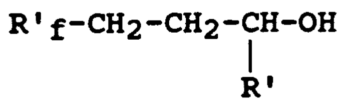

worin $R'_f$ ein Perfluoralkylrest mit 5 bis 13 C-Atomen und R' ein Alkylrest mit 1 bis 5 C-Atomen oder H ist; vergleiche Spalte 2, Reaktionsgleichung (c) in Verbindung mit Spalte 1, Zeilen 27 bis 30 und Beispiel 17. Die primären fluorhaltigen Alkohole werden hergestellt durch Umsetzung von Perfluoralkylethylenen der Formel $R'_f$-$CH = CH_2$, worin $R'_f$ die angegebene Bedeutung hat, mit dem primären Alkohol Methanol und die sekundären fluorhaltigen Alkohole durch Umsetzung der genannten Perfluoralkylethylene mit einem aliphatischen primären Alkohol aus der Gruppe Ethanol, Propanol, Butanol oder Pentanol. Die Umsetzung wird in Gegenwart eines Radikalinitiators und unter Verwendung eines mehr oder weniger großen Überschusses an primärem Alkohol durchgeführt. Dieses Verfahren läßt insbesondere bezüglich Ausbeute zu wünschen übrig.

Es wurde nun gefunden, daß die Umsetzung von Perfluoralkylethylenen mit einem aliphatischen Alkohol dann zu Fluoralkoholen (tertiären Fluoralkoholen) in hoher Ausbeute führt, wenn man als aliphatischen Alkohol sekundäre Alkohole einsetzt (im Gegensatz zum bekannten Verfahren, bei dem ausschließlich primäre aliphatische Alkohole eingesetzt werden, was zu primären und sekundären Fluoralkoholen führt), und zwar den Isopropylalkohol oder den sekundären Butylalkohol.

Das erfindungsgemäße Verfahren zur Herstellung der eingangs genannten tertiären fluorhaltigen Alkohole ist demnach dadurch gekennzeichnet, daß man Perfluoralkylethylene der nachstehenden Formel 2

$R_f$-$CH = CH_2$ (2)

worin $R_f$ die angegebene Bedeutung hat, mit überschüssigem Isopropanol oder überschüssigem sec.-Butanol in Gegenwart von Radikalinitiatoren umsetzt.

Das erfindungsgemäße Verfahren beruht also auf den folgenden Reaktionsgleichungen 1 und 2, wobei in Gleichung 1 Isopropanol und in Gleichung 2 sekundäres Butanol der sekundäre Ausgangsalkohol ist:

Reaktionsgleichung 1:

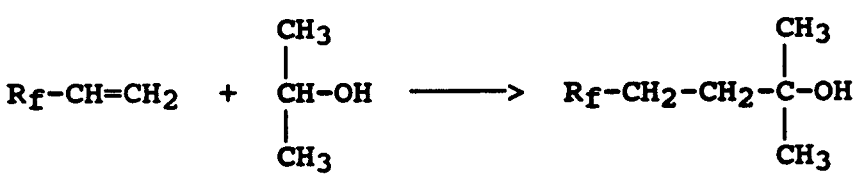

Reaktionsgleichung 2:

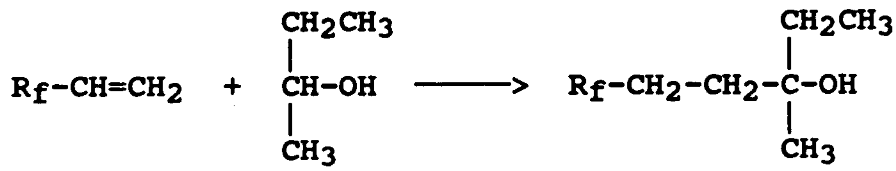

Die beiden Reaktionen verlaufen überraschenderweise praktisch quantitativ, weshalb der angestrebte tertiäre Fluoralkohol in hoher Ausbeute erhalten wird. Es war nicht zu erwarten, daß ein Perfluoralkylethylen gerade mit den in Rede stehenden sekundären Alkoholen so glatt reagiert.

Isopropanol und sec.-Butylalkohol stellen also die eine und Perfluoralkylethylene der Formel $R_f$-$CH=CH_2$ die andere Reaktionskomponente des erfindungsgemäßen Verfahrens dar.

Die Perfluoralkylgruppe $R_f$ mit 4 bis 20 Kohlenstoffatomen, vorzugsweise 6 bis 16 Kohlenstoffatomen, kann gerade oder verzweigt, gesättigt oder ungesättigt (mit vorzugsweise 1 bis 3 Doppelbindungen) sein, wobei gesättigt bevorzugt ist. Im Falle einer verzweigten Perfluoralkylgruppe ist die endverzweigte bevorzugt. Beim Perfluoralkylrest handelt es sich häufig um ein Gemisch von Perfluoralkyl mit der genannten Anzahl von C-Atomen, nämlich 4 bis 20 C-Atomen ($C_4F_9$ bis $C_{20}F_{41}$), vorzugsweise 6 bis 16 C-Atomen ($C_6F_{13}$ bis $C_{16}F_{33}$), oder mit 6 bis 12 C-Atomen ($C_6F_{13}$ bis $C_{12}F_{25}$).

Die Reaktionskomponente sekundärer Alkohol wird im Überschuß eingesetzt, das heißt in einer solchen überschüssigen Menge, daß die Reaktionskomponente Perfluoralkylethylen gut gelöst vorliegt. Es wurde festgestellt, daß in Abwesenheit von überschüssigem sekundärem Alkohol Nebenreaktionen ablaufen und damit Nebenprodukte gebildet werden. Die überschüssige Menge an sekundärem Alkohol im Vergleich zum Perfluoralkylethylen ist nicht kritisch und wird mehr von wirtschaftlichen Überlegungen bestimmt. Im allgemeinen wird man auf 1 mol Perfluoralkylethylen mindestens 3 mol sekundären Alkohol einsetzen, vorzugsweise 5 bis 20 mol sekundären Alkohol.

Die erfindungsgemäße Umsetzung wird ferner in Gegenwart von einem Radikale bildenden Initiator (Radikalinitiator) durchgeführt. Die Menge an Radikalinitiator kann innerhalb weiter Grenzen variieren. Sie hängt insbesondere von der thermischen Zerfallsfähigkeit (Halbwertszeit) der Initiatorverbindung und von der Beständigkeit (Lebensdauer) der gebildeten Radikale ab und liegt in der Regel bei 0,0005 bis 0,1 mol pro mol Perfluoralkylethylen, vorzugsweise bei 0,001 bis 0,05 mol pro mol Perfluoralkylethylen. Die Art des Radikalinitiators ist nicht kritisch. So können anorganische oder organische Initiatoren eingesetzt werden. Dabei wird man in der Regel solche Verbindungen wählen, die bei thermischer Einwirkung vorteilhafte Zerfallserscheinungen aufweisen und reaktionsfähige Radikale liefern. Als Vertreter für anorganische Radikalinitiatoren seien Peroxosäuren, Peroxoborate, Peroxocarbonate, Peroxophosphate und Peroxodisulfate genannt. Als Vertreter für organische Radikalinitiatoren seien die organischen Peroxide (zum Beispiel Dialkylperoxide, Diacylperoxide, Persäuren und Perester) und die Azoverbindungen genannt. Organische Radikalinitiatoren sind bevorzugt. Als organische Peroxide seien im einzelnen genannt: Di-tert.-butylperoxid, tert.-Butylhydroperoxid, tert.-Butylperbenzoat, Benzoylperoxid, Diacetylperoxid und Lauroylperoxid. Als Azoverbindungen seien im einzelnen genannt: Azobisisobutyronitril, 2.2'-Azobis(2-methylbutyronitril) 2.2'-Azobis(2.4-dimethylvaleronitril), Azobisisobutyramidin und 4.4'-Azobis(4-cyanopentansäure).

Die erfindungsgemäße Umsetzung wird ohne Verwendung eines eigenen Lösungsmittels und in einem im wesentlichen wasserfreien Medium durchgeführt. Die Anwesenheit einer geringen Wassermenge, das heißt Wasser bis zu etwa 5 Gew.-%, bezogen auf die Gewichtssumme, aus $R_f$-$CH=CH_2$-Produkt, sekundärem Alkohol und Radikalinitiator, stört im allgemeinen nicht, so daß zum Beispiel auch technisches Isopropanol eingesetzt werden kann. Die Reaktionstemperatur liegt vorteilhafterweise im Bereich von 50 bis 160 °C, vorzugsweise 80 bis 140 °C. Bei einer Temperatur unter 50 °C verläuft die Umsetzung nur sehr langsam und bei über 160 °C treten bereits sehr hohe Drücke auf. Es versteht sich von selbst, daß der eingesetzte Radikalinitiator ein solcher ist, dessen Zerfallstemperatur im genannten Temperaturbereich liegt, was für viele der üblichen Radikalinitiatoren zutrifft, die zudem eine annehmbare Halbwertszeit besitzen. Man wird also eine vorteilhafte Abstimmung zwischen Radikalinitiator und Reaktionstemperatur treffen. Nachdem der Siedepunkt von Isopropanol 80 °C und jener vom sekundären Butanol 100 °C ist, wird man die Reaktion je nach eingesetztem Radikalinitiator drucklos oder unter Druck durchführen. Die Reaktion kann ferner kontinuierlich oder diskontinuierlich durchgeführt werden. Das Ende der Reaktion ist erreicht, wenn kein oder praktisch kein Perfluoralkylethylen mehr festgestellt wird.

Zur Durchführung des erfindungsgemäßen Verfahrens wird man also vorzugsweise so vorgehen, daß

man den sekundären Alkohol, Isopropanol oder sec.-Butanol, die $R_f$-CH = $CH_2$-Verbindung und den Radikalinitiator in den angegebenen Mengen in einem Reaktionsgefäß oder Autoklaven vorlegt, worauf man das Reaktionsgemisch unter Rühren auf Reaktionstemperatur erhitzt und bei dieser Temperatur bis zum Ende der Umsetzung hält. Bei einer weiteren bevorzugten Ausführungsform des erfindungsgemäßen Verfahrens wird man so vorgehen, daß man nur den sekundären Alkohol und das Perfluoralkylethylen-Produkt vorlegt und auf Reaktionstemperatur erhitzt, das heißt auf mindestens jene Temperatur, bei der die gewählte Initiatorverbindung zerfällt. Nachdem diese Temperatur vorliegt, wird die Initiatorverbindung in Form einer mehr oder weniger verdünnten Lösung im eingesetzten sekundären Alkohol in der Menge zudosiert, wie sie in der Reaktionsmischung verbraucht wird. Diese Vorgangsweise wird man unter Umständen aus sicherheitstechnischen Überlegungen vorziehen, weil sie eine niedrigere stationäre Radikalkonzentration gewährtleistet. Das Zudosieren der Radikalinitiatorlösung wird man kontinuierlich oder portionsweise durchführen, zum Beispiel mit Hilfe einer Dosierpumpe. Die Reaktionszeit der erfindungsgemäßen Umsetzung hängt vor allem vom eingesetzten Radikalinitiator und der Reaktionstemperatur ab und liegt in der Regel bei 3 bis 15 Stunden.

Das erfindungsgemäße Verfahren liefert den angestrebten tertiären Fluoralkohol in Form einer Lösung im überschüssig eingesetzten sekundären Alkohol. Da die Umsetzung zum abgestrebten Produkt praktisch quantitativ verläuft und dabei keine nennenswerten Nebenprodukte entstehen, ist eine Isolierung des tertiären Fluoralkohols aus der genannten Lösung oft gar nicht erforderlich; der tertiäre Fluoralkohol stellt vielmehr bereits in dieser Form das angestrebte Produkt dar. Sollte seine Isolierung gewünscht werden, wird man den überschüssigen Alkohol einfach destillativ abtrennen. Dabei wird in der Regel auch gegebenenfalls noch anwesende organische Initiatorverbindung abdestilliert. Das Abtrennen von Initiatorverbindung kann auch zum Beispiel durch Waschen erreicht werden.

Das erfindungsgemäße Verfahren weist eine Reihe von Vorteilen auf. Der angestrebte tertiäre Fluoralkohol wird in hoher Ausbeute und hoher Reinheit erhalten. Das Verfahren ist einfach und wirtschaftlich. Es stellt also eine vorteilhafte Methode dar zur Umwandlung von Perfluoralkylethylenen in tertiäre Fluoralkohole. Die nach dem erfindungsgemäßen Verfahren erhaltenen tertiären Fluoralkohole stellen neue, hoch reaktive Fluorverbindungen dar.

Die Erfindung wird nun an Beispielen noch näher erläutert.

Beispiel 1

Ansatz:
200,00 g (0,375 mol) $R_f$-CH = $CH_2$, $R_f$ = $C_6F_{13}$ bis $C_{12}F_{25}$
338,00 g (5,630 mol) Isopropanol
3,60 g (0,015 mol) Benzoylperoxid
Molares Verhältnis: 1 zu 15 zu 0,04.

Durchführung:
Das $R_f$-CH = $CH_2$-Produkt und das Isopropanol wurden in einem mit Rührer, Rückflußkühler, Thermometer und Tropftrichter ausgestatteten Reaktionsgefäß vorgelegt. Nach dem Spülen mit Stickstoff wurde die Mischung unter Rühren auf 80 °C erhitzt (Siedepunkt von Isopropanol). In die so erhitzte Mischung wurde das Benzoylperoxid, gelöst in 100 g Isopropanol, in einer Zeit von 7 Stunden unter Rühren zugetropft. Nach Beendigung der langsamen Benzoylperoxid-Zugabe wurde die Mischung zur Nachreaktion noch eine weitere Stunde bei 80 °C unter Rühren gehalten. Die Isolierung des gebildeten tertiären Fluoralkohols zwecks Ermittlung der Ausbeute erfolgte hier und in allen weiteren Beispielen durch Abdestillieren des überschüssigen sekundären Alkohols und gegebenenfalls ein- oder mehrmaliges Waschen mit Wasser oder ein schwach alkalischen wäßrigen Lösung.

Ergebnis:
Der erhaltene tertiäre Fluoralkohol war ein bei Raumtemperatur farbloses, kristallines wachsartiges Produkt. Ausbeute: 94 % der Theorie.

Beispiel 2

Ansatz:
50,00 g (0,112 mol) $C_8F_{17}$-CH = $CH_2$
134,70 g (2,240 mol) Isopropanol
0,25 g (0,0017 mol) Di-tert.-butylperoxid
Molares Verhältnis: 1 zu 20 zu 0,015.

Durchführung:

Das $C_8F_{17}$-$CH=CH_2$-Produkt, das Isopropanol und das Peroxid wurden in einem mit Rührer ausgestatteten Autoklaven vorgelegt. Nach dem Spülen mit Stickstoff wurde die Mischung unter Rühren auf 140 °C erhitzt und 5 Stunden lang bei dieser Temperatur unter Rühren gehalten. Der Druck im Autoklaven lag bei 0,8 MPa. Nach der genannten Reaktionszeit war alles Perfluoralkylethylen umgesetzt.

Ergebnis:

Der erhaltene tertiäre Fluoralkohol war ein bei Raumtemperatur farbloses, kristallines wachsartiges Produkt. Ausbeute: 96 % der Theorie.

Beispiel 3

Ansatz:

50,00 g (0,094 mol) $R_f$-$CH=CH_2$, $R_f$ = $C_6F_{13}$ bis $C_{12}F_{25}$
32,40 g (0,540 mol) technisches Isopropanol
1,20 g (0,0086 mol) Di-tert.-butylperoxid
Molares Verhältnis: 1 zu 5 zu 0,08.

Durchführung:

Das $R_f$-$CH=CH_2$-Produkt, Isopropanol und das Peroxid wurden im Autoklaven von Beispiel 2 vorgelegt. Nach dem Spülen mit Stickstoff wurde die Mischung unter Rühren auf 125 °C erhitzt und 10 Stunden lang bei dieser Temperatur unter Rühren gehalten. Der Druck im Autoklaven lag bei 0,5 MPa. Da nach dieser Reaktionszeit die Umsetzung noch nicht beendet war, wurde die Mischung noch weitere 3 Stunden bei der Temperatur von 140 °C unter Rühren gehalten.

Ergebnis:

Wie in Beispiel 2, mit einer Ausbeute von 89 % der Theorie.

Beispiel 4

Ansatz:

200,00 g (0,375 mol) $R_f$-$CH=CH_2$, $R_f$ = $C_6F_{13}$ bis $C_{12}F_{25}$
338,00 g (5,630 mol) Isopropanol
3,84 g (0,0168 mol) Ammoniumperoxodisulfat
Molares Verhältnis: 1 zu 15 zu 0,045.

Durchführung:

Wie in Beispiel 1, mit dem Unterschied, daß in die auf 80 °C erhitzte Mischung das Ammoniumperoxodisulfat, gelöst in 5 g Wasser und 5 g Isopropanol, in einer Zeit von 5 Stunden zugetropft und die Mischung anschließend noch weitere 2 Stunden bei 80 °C zur Nachreaktion gehalten wurde.

Ergebnis:

Wie in Beispiel 1, mit einer Ausbeute von 90 % der Theorie.

Beispiel 5

Ansatz:

200,00 g (0,375 mol) $R_f$-$CH=CH_2$, $R_f$ = $C_6F_{13}$ bis $C_{12}F_{25}$
338,00 g (5,630 mol) Isopropanol
1,08 g (0,0056 mol) Azobisisobutyronitril
Molares Verhältnis: 1 zu 15 zu 0,015.

Durchführung:

Wie in Beispiel 1, mit dem Unterschied, daß in die auf 80 °C erhitzte Mischung das Azobisisobutyronitril, gelöst in 80 g Isopropanol, in einer Zeit von 7 Stunden zugetropft und die Mischung anschließend noch weitere 5 Stunden bei 80 °C zur Nachreaktion gehalten wurde.

Ergebnis:

Wie in Beispiel 1, mit einer Ausbeute von 93 % der Theorie.

Beispiel 6

Ansatz:

200,00 g (0,375 mol) $R_f$-$CH=CH_2$, $R_f$ = $C_6F_{13}$ bis $C_{12}F_{25}$
338,00 g (5,630 mol) Isopropanol
0,27 g (0,0019 mol) Di-tert.-butylperoxid

Molares Verhältnis: 1 zu 15 zu 0,005.

Durchführung:

Das $R_f$-CH = $CH_2$-Produkt und das Isopropanol wurden im Autoklaven von Beispiel 2 vorgelegt. Nach dem Spülen mit Stickstoff wurde die Mischung unter Rühren auf 135 °C erhitzt. In die so erhitzte Mischung wurde das Di-tert.-butylperoxid, gelöst in 80 g Isopropanol, in einer Zeit von 4 Stunden mit Hilfe einer Dosierpumpe unter Rühren zugetropft. Der Druck im Autoklaven lag bei 0,7 MPa. Nach Beendigung der langsamen Peroxid-Zugabe wurde die Mischung noch weitere 5 Stunden bei 140 °C unter Rühren zur Nachreaktion gehalten.

Ergebnis:

Wie in Beispiel 2, mit einer Ausbeute von 95 % der Theorie.

**Patentansprüche**

1. Verfahren zur Herstellung von tertiären fluorhaltigen Alkoholen der nachstehenden Formel 1

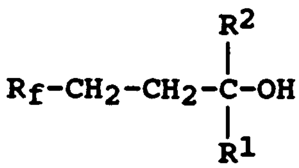

(1)

worin $R_f$ ein Perfluoralkylrest mit 4 bis 20 C-Atomen ist, $R^1$ $CH_3$ ist und $R^2$ $CH_3$ oder $C_2H_5$ ist, dadurch gekennzeichnet, daß man Perfluoralkylethylene der nachstehenden Formel 2

$R_f$-CH = $CH_2$ (2)

worin $R_f$ die angegebene Bedeutung hat, mit überschüssigem Isopropanol oder überschüssigem sec.-Butanol in Gegenwart von Radikalinitiatoren umsetzt.

2. Verfahren nach Anspruch 1, dadurch gekennzeichnet, daß man mindestens 3 mol Isopropanol oder sec.-Butanol pro mol Perfluoralkylethylen einsetzt.

3. Verfahren nach Anspruch 1, dadurch gekennzeichnet, daß man 5 bis 20 mol Isopropanol oder sec.-Butanol pro mol Perfluoralkylethylen einsetzt.

4. Verfahren nach einem der Ansprüche 1 bis 3, dadurch gekennzeichnet, daß man die Umsetzung bei einer Temperatur von 50 bis 160 °C durchführt.

5. Verfahren nach einem der Ansprüche 1 bis 3, dadurch gekennzeichnet, daß man die Umsetzung bei einer Temperatur von 80 bis 140 °C durchführt.

6. Verfahren nach einem oder mehreren der Ansprüche 1 bis 5, dadurch gekennzeichnet, daß man die Radikalinitiatoren in einer Menge von 0,001 bis 0,05 mol pro mol Perfluoralkylethylen einsetzt.

7. Verfahren nach einem oder mehreren der Ansprüche 1 bis 6, dadurch gekennzeichnet, daß man einen organischen Radikalinitiator aus der Gruppe der Dialkylperoxide, Diacylperoxide, Persäuren, Perester und Azoverbindungen einsetzt.

8. Verfahren nach einem oder mehreren der Ansprüche 1 bis 7, dadurch gekennzeichnet, daß Perfluoralkylethylen-Verbindungen eingesetzt werden, deren Perfluoralkylgruppe 6 bis 16 Kohlenstoffatome aufweist.

Europäisches Patentamt

# EUROPÄISCHER RECHERCHENBERICHT

Nummer der Anmeldung

EP 91 11 4005

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,Y | US-A-3532659 (ROBERT BONNER HAGER ET AL.) * Spalte 1, Zeile 27-28; Spalte 2, Gleichung c * | 1-4, 6-8 | C07C31/38<br>C07C29/44 |
| Y | EP-A-163533 (MONTEFLUOS) * Seite 5, Beispiel 3; Ansprüche 1-3 * | 1-4, 6-8 | |
| A | CHEMICAL ABSTRACTS, vol. 93, no. 17, 27 Oktober 1980 Columbus, Ohio, USA R.A. Zamyslov et al.: "Radical telomerisation of 3,3,3-trifluoro-1-propene with 2-propanol" Seite 589; rechte Spalte; ref. no. 167229P & ZH ORG KHIM, 1980, 16[5], 897-901 * Zusammenfassung * | 1 | |
| A | World Patent Index database, Derwent Publ. Ltd London, GB. Accession Nr. 68-36262Q Derwent week 6800 & JP-B-44-021348 (Daikin Kogyo) | 1 | |
| A | FR-A-1065347 (STANDARD OIL DEVELOPMENT) * Ansprüche 1-3 * | 1 | **RECHERCHIERTE SACHGEBIETE (Int. Cl.5 )**<br>C07C |

Der vorliegende Recherchenbericht wurde für alle Patentansprüche erstellt

| Recherchenort | Abschlußdatum der Recherche | Prüfer |
|---|---|---|
| BERLIN | 05 DEZEMBER 1991 | PROBERT,C |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)